# EUROPEAN PATENT SPECIFICATION

(11) **EP 0 621 870 B1**
(45) Date of publication and mention of the grant of the patent: **07.05.1997**
(21) Application number: 93902104.4
(22) Date of filing: 07.01.1993
(51) Int. Cl.: C07K 14/81, C12N 15/15, A61K 38/55

(54) **A HUMAN KUNITZ-TYPE PROTEASE INHIBITOR VARIANT**
INHIBITORVARIANTE DER MENSCHLICHEN PROTEASE VOM KUNITZ-TYP
VARIANT INHIBITEUR DE LA PROTEASE HUMAINE DE TYPE KUNITZ

(30) Priority: 07.01.1992 WO PCT/DK92/00004
(43) Date of publication of application: 02.11.1994
(73) Proprietor: NOVO NORDISK A/S, 2880 Bagsvaerd (DK)
(72) Inventor: NORRIS, Fanny, DK-2900 Hellerup (DK); NORRIS, Kjeld, DK-2900 Hellerup (DK); BJ RN, S ren, Erik, DK-2800 Lyngby (DK); PETERSEN, Lars, Christian, DK-2970 H rsholm (DK); OLSEN, Ole, Hvilsted, DK-2700 Br nsh j (DK)
(74) Representative: Christiansen, Ejvind
(86) International application number: DK9300003
(87) International publication number: WO9314120

(56) References cited:
- US-A- 5 106 833
- Nature, Volume 338, April 1989, THOMAS J. GIRARD et al., "Functional significance of the Kunitz-type inhibitory domains of lipoprotein-associated coagulation inhibitor".

## Description

### FIELD OF INVENTION

The present invention relates to a variant of a human Kunitz-type protease inhibitor domain, DNA encoding the variant, a method of producing the variant and a pharmaceutical composition containing the variant.

### BACKGROUND OF THE INVENTION

Polymorphonuclear leukocytes (neutrophils or PMNs) and mononuclear phagocytes (monocytes) play an important part in tissue injury, infection, acute and chronic inflammation and wound healing. The cells migrate from the blood to the site of inflammation and, following appropriate stimulation, they release oxidant compounds (O₂·, O₂⁻, H₂O₂ and HOCl) as well as granules containing a variety of proteolytic enzymes. The secretory granules contain, i.a., alkaline phosphatase, metalloproteinases such as gelatinase and collagenase and serine proteases such as neutrophil elastase, cathepsin G and proteinase 3.

Latent metalloproteinases are released together with tissue inhibitor of metalloproteinase (TIMP). The activation mechanism has not been fully elucidated, but it is likely that oxidation of thiol groups and/or proteolysis play a part in the process. Also, free metalloproteinase activity is dependent on inactivation of TIMP.

In the azurophil granules of the leukocytes, the serine proteases neutrophil elastase, cathepsin G and proteinase-3 are packed as active enzymes complexed with glucosaminoglycans. These complexes are inactive but dissociate on secretion to release the active enzymes. To neutralise the protease activity, large amounts of the inhibitors α₁-proteinase inhibitor (α₁-PI) and α₁-chymotrypsin inhibitor (α₁-ChI) are found in plasma. However, the PMNs are able to inactivate the inhibitors locally.

Thus, α₁-PI which is the most important inhibitor of neutrophil elastase is sensitive to oxidation at the reactive centre (Met-358) by oxygen metabolites produced by triggered PMNs. This reduces the affinity of α₁-PI for neutrophil elastase by approximately 2000 times.

After local neutralisation of α₁-PI, the elastase is able to degrade a number of inhibitors of other proteolytic enzymes. Elastase cleaves α₁-ChI and thereby promotes cathepsin G activity. It also cleaves TIMP, resulting in tissue degradation by metalloproteinases. Furthermore, elastase cleaves antithrombin III and heparin cofactor II, and tissue factor pathway inhibitor (TFPI) which probably promotes clot formation. On the other hand, the ability of neutrophil elastase to degrade coagulation factors is assumed to have the opposite effect so that the total effect of elastase is unclear. The effect of neutrophil elastase on fibrinolysis is less ambiguous. Fibrinolytic activity increases when the elastase cleaves the plasminogen activator inhibitor and the α₂ plasmin inhibitor. Besides, both of these inhibitors are oxidated and inactivated by O₂ metabolites.

PMNs contain large quantities of serine proteases, and about 200 mg of each of the leukocyte proteases are released daily to deal with invasive agents in the body. Acute inflammation leads to a many-fold increase in the amount of enzyme released. Under normal conditions, proteolysis is kept at an acceptably low level by large amounts of the inhibitors α₁-PI, α₁-ChI and α₂ macroglobulin. There is some indication, however, that a number of chronic diseases is caused by pathological proteolysis due to overstimulation of the PMNs, for instance caused by autoimmune response, chronic infection, tobacco smoke or other irritants, etc.

Aprotinin (bovine pancreatic trypsin inhibitor) is known to inhibit various serine proteases, including trypsin, chymotrypsin, plasmin and kallikrein, and is used therapeutically in the treatment of acute pancreatitis, various states of shock syndrome, hyperfibrinolytic haemorrhage and myocardial infarction (cf., for instance, J.E. Trapnell et al, Brit. J. Surg. 61, 1974, p. 177; J. McMichan et al., Circulatory shock 9, 1982, p. 107; L.M. Auer et al., Acta Neurochir. 49, 1979, p. 207; G. Sher, Am. J. Obstet. Gynecol. 129, 1977, p. 164; and B. Schneider, Artzneim.-Forsch. 26, 1976, p. 1606). Administration of aprotinin in high doses significantly reduces blood loss in connection with cardiac surgery, including cardiopulmonary bypass operations (cf., for instance, B.P. Bidstrup et al., J. Thorac. Cardiovasc. Surg. 97, 1989, pp. 364-372; W. van Oeveren et al., Ann. Thorac. Surg. 44, 1987, pp. 640-645). It has previously been demonstrated (cf. H.R. Wenzel and H. Tschesche, Angew. Chem. Internat. Ed. 20, 1981, p. 295) that certain aprotinin analogues, e.g. aprotinin(1-58, Vall5) exhibits a relatively high selectivity for granulocyte elastase and an inhibitory effect on collagenase, aprotinin (1-58, Alal5) has a weak effect on elastase, while aprotinin (3-58, Argl5, Ala17, Ser42) exhibits an excellent plasma kallikrein inhibitory effect (cf. WO 89/10374).

However, when administered in vivo, aprotinin has been found to have a nephrotoxic effect in rats, rabbits and dogs after repeated injections of relatively high doses of aprotinin (Bayer, Trasvlol. Inhibitor of Proteinase; E. Glaser et al. in "Verhandlungen der Deutschen Gesellschaft für Innere Medizin, 78. Kongress", Bergmann, München, 1972, pp. 1612-1614). The nephrotoxicity (i.a. appearing in the form of lesions) observed for aprotinin might be ascribed to the accumulation of aprotinin in the proximal tubulus cells of the kidneys as a result of the high positive net charge of aprotinin which causes it to be bound to the negatively charged surfaces of the tubuli.. This nephrotoxicity makes aprotinin less suitable for clinical purposes, in particular those requiring administration of large doses of the inhibitor (such as cardiopulmonary bypass operations). Besides, aprotinin is a bovine protein which may therefore contain one or more epitopes which may give rise to an undesirable immune response on administration of aprotinin to humans.

It is therefore an object of the present invention to identify human protease inhibitors of the same type as aprotinin (i.e. Kunitz-type inhibitors) with a similar inhibitor profile or modified to exhibit a desired inhibitor profile.

### SUMMARY OF THE INVENTION

The present invention relates to a variant of human Kunitz-type protease inhibitor domain III of tissue factor pathway inhibitor (TFPI), the variant comprising the following amino acid sequence wherein X¹ represents H or 1-5 naturally occurring amino acid residues except Cys, X²-X¹⁵ each independently represents a naturally occurring amino acid residue except Cys, and X¹⁶ represents OH or 1-5 naturally occurring amino acid residues except Cys, with the proviso that at least one of the amino acid residues X¹-X¹⁶ is different from the corresponding amino acid residue of the native sequence.

In the present context, the term "naturally occurring amino acid residue" is intended to indicate any one of the 20 commonly occurring amino acids, i.e. Ala, Val, Leu, Ile Pro, Phe, Trp, Met, Gly, Ser, Thr, Cys, Tyr, Asn, Gln, Asp, Glu, Lys, Arg and His.

TFPI, also known as extrinsic pathway inhibitor (EPI) or lipoprotein associated coagulation inhibitor (LACI), has been isolated by Broze et al. (Proc. Natl. Acad. Sci. USA 84, 1987, pp. 1886-1890 and EP 300 988) and the gene coding for the protein has been cloned, cf. EP 318 451. Analysis of the secondary structure of the protein has shown that the protein has three Kunitz-type inhibitor domains, from amino acid 22 to amino acid 79 (I), from amino acid 93 to amino acid 150 (II) and from amino acid 185 to amino acid 242 (III). Kunitz-type domain I of TFPI has been shown to bind TF/FVIIa, while Kunitz-type domain II has been shown to bind to FXa (Girard et al., Nature 338, 1989, pp. 518-520).

By substituting one or more amino acids in one or more of the positions indicated above, it may be possible to change the inhibitor profile of TFPI Kunitz-type domain III so that it preferentially inhibits neutrophil elastase, cathepsin G and/or proteinase-3. Furthermore, it may be possible to construct variants which specifically inhibit enzymes involved in coagulation or fibrinolysis (e.g. plasmin or plasma kallikrein) or the complement cascade.

One advantage of TFPI Kunitz-type domain III is that it has a negative net charge as opposed to aprotinin which, as indicated above, has a strongly positive net charge. It is therefore possible to construct variants of the invention with a lower positive net charge than aprotinin, thereby reducing the risk of kidney damage on administration of large doses of the variants. Another advantage is that, contrary to aprotinin, it is a human protein (fragment) so that undesired immunological reactions on administration to humans are significantly reduced.

### DETAILED DISCLOSURE OF THE INVENTION

Examples of preferred variants of Kunitz-type domain III of TFPI are variants wherein X¹ is Gly-Pro; or wherein X² is an amino acid residue selected from the group consisting of Ala, Arg, Thr, Asp, Pro, Glu, Lys, Gln, Ser, Ile and Val, in particular wherein X² is Thr or Arg; or wherein X³ is an amino acid residue selected from the group consisting of Pro, Thr, Leu, Arg, Val and Ile, in particular wherein X³ is Pro or Leu; or wherein X⁴ is an amino acid residue selected from the group consisting of Lys, Arg, Val, Thr, Ile, Leu, Phe, Gly, Ser, Met, Trp, Tyr, Gln, Asn and Ala, in particular wherein X⁴ is Lys, Val, Leu, Ile, Thr, Met, Gln or Arg; or wherein X⁵ is an amino acid residue selected from the group consisting of Ala, Gly, Thr, Arg, Phe, Gln and Asp, in particular wherein X⁵ is Ala, Thr, Asp or Gly; or wherein X⁶ is an amino acid residue selected from the group consisting of Arg, Ala, Lys, Leu, Gly, His, Ser, Asp, Gln, Glu, Val, Thr, Tyr, Phe, Asn, Ile and Met, in particular wherein X⁶ is Arg, Phe, Ala, Asn, Leu or Tyr; or wherein X⁷ is an amino acid residue selected from the group consisting of Ile, Met, Gln, Glu, Thr, Leu, Val and Phe, in particular wherein X⁷ is Ile or Glu; or wherein X⁸ is an amino acid residue selected from the group consisting of Ile, Thr, Leu, Asn, Lys, Ser, Gln, Glu, Arg, Pro and Phe, in particular wherein X⁸ is Ile or Asn; or wherein X⁹ is an amino acid residue selected from the group consisting of Arg, Ser, Ala, Gln, Lys and Leu, in particular wherein X⁹ is Arg; or wherein X¹⁰ is an amino acid residue selected from the group consisting of Gln, Pro, Phe, Ile Lys, Trp, Ala, Thr, Leu, Ser, Tyr, His, Asp, Met, Arg and Val, in particular wherein X¹⁰ is Val or Lys; or wherein X¹¹ is Ser or Gly; or wherein X¹² is an amino acid residue selected from the group consisting of Gly, Met, Gln, Glu, Leu, Arg, Lys, Pro and Asn, in particular wherein X¹² is Arg or Glu; or wherein X¹³ is Ala or Gly; or wherein X¹⁴ is an amino acid residue selected from the group consisting of Lys, Asn and Asp, in particular wherein X¹⁴ is Lys or Asn; or wherein X¹⁵ is an amino acid residue selected from the group consisting of Val, Tyr, Asp, Glu, Thr, Gly, Leu, Ser, Ile, Gln, His, Asn, Pro, Phe, Met, Ala, Arg, Trp and Lys, in particular wherein X¹⁵ is Lys or Thr; or wherein X¹⁶ is Gly. In a preferred embodiment, X¹ is Lys-Pro and X¹⁶ is Gly, while X²-X¹⁵ are as defined above.

Variants of TFPI Kunitz-type domain III of the invention should preferably not contain a Met residue in the protease binding region (i.e. the amino acid residues represented by X³-X¹⁴). By analogy to α1-PI described above, a Met residue in any one of these positions would make the inhibitor sensitive to oxidative inactivation by oxygen metabolites produced by PMNs, and conversely, lack of a Met residue in these positions should render the inhibitor more stable in the presence of such oxygen metabolites.

A currently preferred variant of the invention is one in which one or more of the amino acid residues located at the protease-binding site of the Kunitz domain (i.e. one or more of X³-X¹⁴ corresponding to positions 13, 15, 16, 17, 18, 19, 20, 34, 39, 40, 41 and 46 of aprotinin) are substituted to the amino acids present in the same positions of native aprotinin. This variant comprises the following amino acid sequence

In another aspect, the invention relates to a DNA construct encoding a human Kunitz-type inhibitor domain variant according to the invention. The DNA construct of the invention may be prepared synthetically by established standard methods, e.g. the phosphoamidite method described by S.L. Beaucage and M.H. Caruthers, Tetrahedron Letters 22, 1981, pp. 1859-1869, or the method described by Matthes et al., EMBO Journal 3, 1984, pp. 801-805. According to the phosphoamidite method, oligonucleotides are synthesized, e.g. in an automatic DNA synthesizer, purified, annealed, ligated and cloned in suitable vectors.

Alternatively, it is possible to use genomic or cDNA coding for TFPI Kunitz-type domain III (e.g. obtained by screening a genomic or cDNA library for DNA coding for TFPI using synthetic oligonucleotide probes and isolating the DNA sequence coding for domain III therefrom). The DNA sequence is modified at one or more sites corresponding to the site(s) at which it is desired to introduce amino acid substitutions, e.g. by site-directed mutagenesis using synthetic oligonucleotides encoding the desired amino acid sequence for homologous recombination in accordance with well-known procedures.

In a still further aspect, the invention relates to a recombinant expression vector which comprises a DNA construct of the invention. The recombinant expression vector may be any vector which may conveniently be subjected to recombinant DNA procedures, and the choice of vector will often depend on the host cell into which it is to be introduced. Thus, the vector may be an autonomously replicating vector, i.e. a vector which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

In the vector, the DNA sequence encoding the TFPI Kunitz-type domain III variant of the invention should be operably connected to a suitable promoter sequence. The promoter may be any DNA sequence which shows transcriptional activity in the host cell of choice and may be derived from genes encoding proteins either homologous or heterologous to the host cell. Examples of suitable promoters for directing the transcription of the DNA encoding the TFPI Kunitz-type domain III variant of the invention in mammalian cells are the SV 40 promoter (Subramani et al., Mol. Cell Biol. 1, 1981, pp. 854-864), the MT-1 (metallothionein gene) promoter (Palmiter et al., Science 222, 1983, pp. 809-814) or the adenovirus 2 major late promoter. Suitable promoters for use in yeast host cells include promoters from yeast glycolytic genes (Hitzeman et al., J. Biol. Chem. 255, 1980, pp. 12073-12080; Alber and Kawasaki, J. Mol. Appl. Gen. 1, 1982, pp. 419-434) or alcohol dehydrogenase genes (Young et al., in Genetic Engineering of Microorganisms for Chemicals (Hollaender et al, eds.), Plenum Press, New York, 1982), or the TPI1 (US 4, 599, 311) or ADH2-4c (Russell et al., Nature 304, 1983, pp. 652-654) promoters. Suitable promoters for use in filamentous fungus host cells are, for instance, the ADH3 promoter (McKnight et al., The EMBO J. 4, 1985, pp. 2093-2099) or the tpiA promoter.

The DNA sequence encoding the TFPI Kunitz-type domain III variant of the invention may also be operably connected to a suitable terminator, such as the human growth hormone terminator (Palmiter et al., op. cit.) or (for fungal hosts) the TPI1 (Alber and Kawasaki, op. cit.) or ADH3 (McKnight et al., op. cit.) promoters. The vector may further comprise elements such as polyadenylation signals (e.g. from SV 40 or the adenovirus 5 Elb region), transcriptional enhancer sequences (e.g. the SV 40 enhancer) and translational enhancer sequences (e.g. the ones encoding adenovirus VA RNAs).

The recombinant expression vector of the invention may further comprise a DNA sequence enabling the vector to replicate in the host cell in question. An examples of such a sequence (when the host cell is a mammalian cell) is the SV 40 origin of replication, or (when the host cell is a yeast cell) the yeast plasmid 2µ replication genes REP 1-3 and origin of replication. The vector may also comprise a selectable marker, e.g. a gene the product of which complements a defect in the host cell, such as the gene coding for dihydrofolate reductase (DHFR) or one which confers resistance to a drug, e.g. neomycin, hygromycin or methotrexate, or the Schizosaccharomyces pombe TPI gene (described by P.R. Russell, Gene 40, 1985, pp. 125-130.

The procedures used to ligate the DNA sequences coding for the TFPI Kunitz-type domain III variant of the invention, the promoter and the terminator, respectively, and to insert them into suitable vectors containing the information necessary for replication, are well known to persons skilled in the art (cf., for instance, Sambrook et al., Molecular Cloning: A Laboratory Manual, Cold Spring Harbor, New York, 1989).

The host cell into which the expression vector of the invention is introduced may be any cell which is capable of producing the TFPI Kunitz-type domain III variant of the invention and is preferably a eukaryotic cell, such as a mammalian, yeast or fungal cell.

The yeast organism used as the host cell according to the invention may be any yeast organism which, on cultivation, produces large quantities of the TFPI Kunitz-type domain III variant of the invention. Examples of suitable yeast organisms are strains of the yeast species Saccharomyces cerevisiae, Saccharomyces kluyveri, Schizosaccharomyces pombe or Saccharomyces uvarum. The transformation of yeast cells may for instance be effected by protoplast formation followed by transformation in a manner known per se.

Examples of suitable mammalian cell lines are the COS (ATCC CRL 1650), BHK (ATCC CRL 1632, ATCC CCL 10) or CHO (ATCC CCL 61) cell lines. Methods of transfecting mammalian cells and expressing DNA sequences introduced in the cells are described in e.g. Kaufman and Sharp, J. Mol. Biol. 159, 1982, pp. 601-621; Southern and Berg, J. Mol. Appl. Genet. 1, 1982, pp. 327-341; Loyter et al., Proc. Natl. Acad. Sci. USA 79, 1982, pp. 422-426; Wigler et al., Cell 14, 1978, p. 725; Corsaro and Pearson, Somatic Cell Genetics 7, 1981, p. 603, Graham and van der Eb, Virology 52, 1973, p. 456; and Neumann et al., EMBO J. 1, 1982, pp. 841-845.

Alternatively, fungal cells may be used as host cells of the invention. Examples of suitable fungal cells are cells of filamentous fungi, e.g. Aspergillus spp. or Neurospora spp., in particular strains of Aspergillus oryzae or Aspergillus niger. The use of Aspergillus spp. for the expression of proteins is described in, e.g., EP 238 023.

The present invention further relates to a method of producing a TFPI Kunitz-type domain III variant according to the invention, the method comprising culturing a cell as described above under conditions conducive to the expression of the variant and recovering the resulting variant from the culture.

The medium used to cultivate the cells may be any conventional medium suitable for growing mammalian cells or fungal (including yeast) cells, depending on the choice of host cell. The variant will be secreted by the host cells to the growth medium and may be recovered therefrom by conventional procedures including separating the cells from the medium by centrifugation or filtration, precipitating the proteinaceous components of the supernatant or filtrate by means of a salt, e.g. ammonium sulfate, purification by a variety of chromatographic procedures, e.g. ion exchange chromatography or affinity chromatography, or the like.

The present invention also relates to a pharmaceutical composition comprising a TFPI Kunitz-type domain III variant of the invention together with a pharmaceutically acceptable carrier or excipient. In the composition of the invention, the variant may be formulated by any of the established methods of formulating pharmaceutical compositions, e.g. as described in Remington's Pharmaceutical Sciences, 1985. The composition may typically be in a form suited for systemic injection or infusion and may, as such, be formulated with sterile water or an isotonic saline or glucose solution.

The TFPI Kunitz-type domain III variant of the invention is therefore contemplated to be advantageous to use for the therapeutic applications suggested for native aprotinin or aprotinin analogues with other inhibitor profiles, in particular those which necessitate the use of large aprotinin doses. Therapeutic applications for which the use of the variant of the invention is indicated as a result of its ability to inhibit human serine proteases, e.g. trypsin, plasmin, kallikrein, elastase, cathepsin G and proteinase-3, include (but are not limited to) acute pancreatitis, inflammation, thrombocytopenia, preservation of platelet function, organ preservation, wound healing, shock (including shock lung) and conditions involving hyperfibrinolytic haemorrhage, emphysema, rheumatoid arthritis, adult respiratory distress syndrome, chronic inflammatory bowel disease and psoriasis, in other words diseases presumed to be caused by pathological proteolysis by elastase, cathepsin G and proteinase-3 released from triggered PMNs.

Furthermore, the present invention relates to the use of TFPI Kunitz-type inhibitor domain III or a variant thereof as described above for the preparation of a medicament for the prevention or therapy of diseases or conditions associated with pathological proteolysis by proteases released from overstimulated PMNs. As indicated above, it may be an advantage of administer heparin concurrently with the TFPI Kunitz-type inhibitor domain III or variant.

Apart from the pharmaceutical use indicated above, TFPI Kunitz-type domain II or a variant thereof as specified above may be used to isolate useful natural substances, e.g. proteases or receptors from human material, which bind directly or indirectly to TFPI Kunitz-type domain II, for instance by screening assays or by affinity chromatography.

## Claims

1. A variant of human Kunitz-type protease inhibitor domain III of tissue factor protease inhibitor (TFPI), the variant comprising the following amino acid sequence wherein X¹ represents H or 1-5 naturally occurring amino acid residues except Cys, X²-X¹⁵ each independently represents a naturally occurring amino acid residue, and X¹⁶ represents OH or 1-5 naturally occurring amino acid residues except Cys, with the proviso that at least one of the amino acid residues X¹-X¹⁶ is different from the corresponding amino acid residue of the native sequence, and with the further proviso that X⁴ is not Leu.

2. A variant according to claim 1, wherein X¹ is Gly-Pro.

3. A variant according to claim 1, wherein X² is an amino acid residue selected from the group consisting of Ala, Arg, Thr, Asp, Pro, Glu, Lys, Gln, Ser, Ile and Val.

4. A variant according to claim 3, wherein X² is Thr or Arg.

5. A variant according to claim 1, wherein X³ is an amino acid residue selected from the group consisting of Pro, Thr, Leu, Arg, Val and Ile.

6. A variant according to claim 5, wherein X³ is Pro or Leu.

7. A variant according to claim 1, wherein X⁴ is an amino acid residue selected from the group consisting of Lys, Arg, Val, Thr, Ile, Phe, Gly, Ser, Met, Trp, Tyr, Gln, Asn and Ala.

8. A variant according to claim 7, wherein X⁴ is Lys, Val, Ile, Thr, Met, GIn or Arg.

9. A variant according to claim 1, wherein X⁵ is an amino acid residue selected from the group consisting of Ala, Gly, Thr, Arg, Phe, Gln and Asp.

10. A variant according to claim 9, wherein X⁵ is Ala, Thr, Asp or Gly.

11. A variant according to claim 1, wherein X⁶ is an amino acid residue selected from the group consisting of Arg, Ala, Lys, Leu, Gly, His, Ser, Asp, Gln, Glu, Val, Thr, Tyr, Phe, Asn, Ile and Met.

12. A variant according to claim 11, wherein X⁶ is Arg, Phe, Ala, Asn, Leu or Tyr.

13. A variant according to claim 1, wherein X⁷ is an amino acid residue selected from the group consisting of Ile, Met, Gln, Glu, Thr, Leu, Val and Phe.

14. A variant according to claim 13, wherein X⁷ is Ile or Glu.

15. A variant according to claim 1, wherein X⁸ is an amino acid residue selected from the group consisting of Ile, Thr, Leu, Asn, Lys, Ser, Gln, Glu, Arg, Pro and Phe.

16. A variant according to claim 15, wherein X⁸ is Ile or Asn.

17. A variant according to claim 1, wherein X⁹ is an amino acid residue selected from the group consisting of Arg, Ser, Ala, Gln, Lys and Leu.

18. A variant according to claim 17, wherein X⁹ is Arg.

19. A variant according to claim 1, wherein X¹⁰ is an amino acid residue selected from the group consisting of Gln, Pro, Phe, Ile Lys, Trp, Ala, Thr, Leu, Ser, Tyr, His, Asp, Met; Arg and Val.

20. A variant according to claim 19, wherein X¹⁰ is Val or Lys.

21. A variant according to claim 1, wherein X¹¹ is Ser or Gly.

22. A variant according to claim 1, wherein X¹² is an amino acid residue selected from the group consisting of Gly, Met, Gln, Glu, Leu, Arg, Lys, Pro and Asn.

23. A variant according to claim 22, wherein X¹² is Ala or Leu.

24. A variant according to claim 1, wherein X¹³ is Ala or Gly.

25. A variant according to claim 1, wherein X¹⁴ is an amino acid residue selected from the group consisting of Lys, Asn and Asp.

26. A variant according to claim 25, wherein X¹⁴ is Lys or Asn.

27. A variant according to claim 1, wherein X¹⁵ is an amino acid residue selected from the group consisting of Val, Tyr, Asp, Glu, Thr, Gly, Leu, Ser, Ile, Gln, His, Asn, Pro, Phe, Met, Ala, Arg, Trp and Lys.

28. A variant according to claim 27, wherein X¹⁵ is Lys or Glu.

29. A variant according to claim 1, wherein X¹⁶ is Gly.

30. A variant according to claim 1, wherein X¹ is Gly-Pro and X¹⁶ is Gly.

31. A variant according to claim 1 comprising the following amino acid sequence

32. A DNA construct comprising a DNA sequence encoding a human Kunitz-type protease inhibitor variant according to any of claims 1-31.

33. A recombinant expression vector comprising a DNA construct according to claim 32.

34. A cell containing a DNA construct according to claim 32 or an expression vector according to claim 33.

35. A method of producing a human Kunitz-type protease inhibitor variant according to any of claims 1-31, the method comprising culturing a cell according to claim 34 under conditions conducive to the expression of the protein, and recovering the resulting protein from the culture.

36. A pharmaceutical composition comprising a human Kunitz-type protease inhibitor variant according to any of claims 1-31 and a pharmaceutically acceptable carrier or excipient.

37. A composition according to claim 36 which further comprises heparin.

38. Use of human Kunitz-type protease inhibitor domain III of TFPI or a variant thereof according to any of claims 1-31 for the preparation of a medicament for the prevention or treatment of diseases or conditions associated with pathological proteolysis.

## Patentansprüche

1. Variante der als humaner Protease-Inhibitor des Kunitz-Typs wirkenden Domäne III des Gewebefaktorprotease-Inhibitors (TFPI), wobei die Variante die folgende Aminosäuresequenz umfaßt: wobei X¹ H oder 1-5 natürlich vorkommende Aminosäurereste außer Cys darstellt, X²-X¹⁵ jeweils unabhängig einen natürlich vorkommenden Aminosäurerest darstellen und X¹⁶ OH oder 1-5 natürlich vorkommende Aminosäurereste außer Cys darstellt, mit der Maßgabe, daß sich wenigstens einer der Aminosäurereste X¹-X¹⁶ von dem entsprechenden Aminosäurerest der nativen Sequenz unterscheidet, und mit der weiteren Maßgabe, daß X⁴ nicht Leu ist.

2. Variante gemäß Anspruch 1, wobei X¹ Gly-Pro ist.

3. Variante gemäß Anspruch 1, wobei X² ein Aminosäurerest ist, der aus der Gruppe ausgewählt ist, die aus Ala, Arg, Thr, Asp, Pro, Glu, Lys, Gln, Ser, Ile und Val besteht.

4. Variante gemäß Anspruch 3, wobei X² Thr oder Arg ist.

5. Variante gemäß Anspruch 1, wobei X³ ein Aminosäurerest ist, der aus der Gruppe ausgewählt ist, die aus Pro, Thr, Leu, Arg, Val und Ile besteht.

6. Variante gemäß Anspruch 5, wobei X³ Pro oder Leu ist.

7. Variante gemäß Anspruch 1, wobei X⁴ ein Aminosäurerest ist, der aus der Gruppe ausgewählt ist, die aus Lys, Arg, Val, Thr, Ile, Phe, Gly, Ser, Met, Trp, Tyr, Gln, Asn und Ala besteht.

8. Variante gemäß Anspruch 7, wobei X⁴ Lys, Val, Ile, Thr, Met, Gln oder Arg ist.

9. Variante gemäß Anspruch 1, wobei X⁵ ein Aminosäurerest ist, der aus der Gruppe ausgewählt ist, die aus Ala, Gly, Thr, Arg, Phe, Gln und Asp besteht.

10. Variante gemäß Anspruch 9, wobei X⁵ Ala, Thr, Asp oder Gly ist.

11. Variante gemäß Anspruch 1, wobei X⁶ ein Aminosäurerest ist, der aus der Gruppe ausgewählt ist, die aus Arg, Ala, Lys, Leu, Gly, His, Ser, Asp, Gln, Glu, Val, Thr, Tyr, Phe, Asn, Ile und Met besteht.

12. Variante gemäß Anspruch 11, wobei X⁶ Arg, Phe, Ala, Asn, Leu oder Tyr ist.

13. Variante gemäß Anspruch 1, wobei X⁷ ein Aminosäurerest ist, der aus der Gruppe ausgewählt ist, die aus Ile, Met; GIn, Glu, Thr, Leu, Val und Phe besteht.

14. Variante gemäß Anspruch 13, wobei X⁷ Ile oder Glu ist.

15. Variante gemäß Anspruch 1, wobei X⁸ ein Aminosäurerest ist, der aus der Gruppe ausgewählt ist, die aus Ile, Thr, Leu, Asn, Lys, Ser, Gln, Glu, Arg, Pro und Phe besteht.

16. Variante gemäß Anspruch 15, wobei X⁸ Ile oder Asn ist.

17. Variante gemäß Anspruch 1, wobei X⁹ ein Aminosäurerest ist, der aus der Gruppe ausgewählt ist, die aus Arg, Ser, Ala, Gln, Lys und Leu besteht.

18. Variante gemäß Anspruch 17, wobei X⁹ Arg ist.

19. Variante gemäß Anspruch 1, wobei X¹⁰ ein Aminosäurerest ist, der aus der Gruppe ausgewählt ist, die aus Gln, Pro, Phe, Ile, Lys, Trp, Ala, Thr, Leu, Ser, Tyr, His, Asp, Met, Arg und Val besteht.

20. Variante gemäß Anspruch 19, wobei X¹⁰ Val oder Lys ist.

21. Variante gemäß Anspruch 1, wobei X¹¹ Ser oder Gly ist.

22. Variante gemäß Anspruch 1, wobei X¹² ein Aminosäurerest ist, der aus der Gruppe ausgewählt ist, die aus Gly, Met, Gln, Glu, Leu, Arg, Lys, Pro und Asn besteht.

23. Variante gemäß Anspruch 22, wobei X¹² Ala oder Leu ist.

24. Variante gemäß Anspruch 1, wobei X¹³ Ala oder Gly ist.

25. Variante gemäß Anspruch 1, wobei X¹⁴ ein Aminosäurerest ist, der aus der Gruppe ausgewählt ist, die aus Lys, Asn und Asp besteht.

26. Variante gemäß Anspruch 25, wobei X¹⁴ Lys oder Asn ist.

27. Variante gemäß Anspruch 1, wobei X¹⁵ ein Aminosäurerest ist, der aus der Gruppe ausgewählt ist, die aus Val, Tyr, Asp, Glu, Thr, Gly, Leu, Ser, Ile, Gln, His, Asn, Pro, Phe, Met, Ala, Arg, Trp und Lys besteht.

28. Variante gemäß Anspruch 27, wobei X¹⁵ Lys oder Glu ist.

29. Variante gemäß Anspruch 1, wobei X¹⁶ Gly ist.

30. Variante gemäß Anspruch 1, wobei X¹ Gly-Pro ist und X¹⁶ Gly ist.

31. Variante gemäß Anspruch 1, die die folgende Aminosäuresequenz umfaßt:

32. DNA-Konstrukt, das eine DNA-Sequenz umfaßt, die für eine Variante eines humanen Protease-Inhibitors des Kunitz-Typs gemäß einem der Ansprüche 1-31 codiert.

33. Rekombinanter Expressionsvektor, der ein DNA-Konstrukt gemäß Anspruch 32 umfaßt.

34. Zelle, die ein DNA-Konstrukt gemäß Anspruch 32 oder einen Expressionsvektor gemäß Anspruch 33 enthält.

35. Verfahren zur Herstellung einer Variante eines humanen Protease-Inhibitors des Kunitz-Typs gemäß einem der Ansprüche 1-31, wobei das Verfahren das Kultivieren einer Zelle gemäß Anspruch 34 unter Bedingungen, die der Expression des Proteins förderlich sind, und das Gewinnen des resultierenden Proteins aus der Kultur umfaßt.

36. Pharmazeutische Zusammensetzung, die eine Variante eines humanen Protease-Inhibitors des Kunitz-Typs gemäß einem der Ansprüche 1-31 sowie einen pharmazeutisch annehmbaren Träger oder Arzneimittelhilfsstoff umfaßt.

37. Zusammensetzung gemäß Anspruch 36, die weiterhin Heparin umfaßt.

38. Verwendung der als humaner Protease-Inhibitor des Kunitz-Typs wirkenden Domäne III des TFPI oder einer Variante davon gemäß einem der Ansprüche 1-31 zur Herstellung eines Medikaments zur Vorbeugung oder Behandlung von Krankheiten oder Zuständen, die mit pathologischer Proteolyse verbunden sind.

## Revendications

1. Variant du domaine III inhibiteur de la protéase humaine du type Kunitz de l'inhibiteur de la protéase du facteur tissulaire (TFPI), le variant comprenant la séquence d'acides aminés suivante dans laquelle X¹ représente H ou 1-5 résidus d'acides aminés naturels sauf Cys, X²-X¹⁵ représentent chacun indépendamment un résidu d'acide aminé naturel, et X¹⁶ représente OH ou 1-5 résidus d'acides aminés naturels sauf Cys, à condition qu'au moins l'un des résidus d'acides aminés X¹-X¹⁶ soit différent du résidu d'acide aminé correspondant de la séquence native, sous la condition supplémentaire que X⁴ n'est pas Leu.

2. Variant selon la revendication 1, caractérisé en ce que X¹ est Gly-Pro.

3. Variant selon la revendication 1, caractérisé en ce que X est un résidu d'acide aminé choisi dans le groupe constitué de Ala, Arg, Thr, Asp, Pro, Glu, Lys, Gln, Ser, Ile et Val.

4. Variant selon la revendication 3, caractérisé en ce que X² est Thr ou Arg.

5. Variant selon la revendication 1, caractérisé en ce que X³ est un résidu d'acide aminé choisi dans le groupe constitué de Pro, Thr, Leu, Arg, Val et Ile.

6. Variant selon la revendication 5, caractérisé en ce que X³ est Pro ou Leu.

7. Variant selon la revendication 1, caractérisé en ce que X⁴ est un résidu d'acide aminé choisi dans le groupe constitué de Lys, Arg, Val, Thr, Ile, Phe, Gly, Ser, Met, Trp, Tyr, Gln, Asn et Ala.

8. Variant selon la revendication 7, caractérisé en ce que X⁴ est Lys, Val, Ile, Thr, Met, Gln ou Arg.

9. Variant selon la revendication 1, caractérisé en ce que X⁵ est un résidu d'acide aminé choisi dans le groupe constitué de Ala, Gly, Thr, Arg, Phe, Gln et Asp.

10. Variant selon la revendication 9, caractérisé en ce que X⁵ est Ala, Thr, Asp ou Gly.

11. Variant selon la revendication 1, caractérisé en ce que X⁶ est un résidu d'acide aminé choisi dans le groupe constitué de Arg, Ala, Lys, Leu, Gly, His, Ser, Asp, Gln, Glu, Val, Thr, Tyr, Phe, Asn, Ile et Met.

12. Variant selon la revendication 11, caractérisé en ce que X⁶ est Arg, Phe, Ala, Asn, Leu ou Tyr.

13. Variant selon la revendication 1, caractérisé en ce que X⁷ est un résidu d'acide aminé choisi dans le groupe constitué de Ile, Met, Gln, Glu, Thr, Leu, Val et Phe.

14. Variant selon la revendication 13, caractérisé en ce que X⁷ est Ile ou Glu.

15. Variant selon la revendication 1, caractérisé en ce que X⁸ est un résidu d'acide aminé choisi dans le groupe constitué de Ile, Thr, Leu, Asn, Lys, Ser, Gln, Glu, Arg, Pro et Phe.

16. Variant selon la revendication 15, caractérisé en ce que X⁸ est Ile ou Asn.

17. Variant selon la revendication 1, caractérisé en ce que X⁹ est un résidu d'acide aminé choisi dans le groupe constitué de Arg, Ser, Ala, Gln, Lys et Leu.

18. Variant selon la revendication 17, caractérisé en ce que X⁹ est Arg.

19. Variant selon la revendication 1, caractérisé en ce que X¹⁰ est un résidu d'acide aminé choisi dans le groupe constitué de Gln, Pro, Phe, Ile, Lys, Trp, Ala, Thr, Leu, Ser, Tyr, His, Asp, Met, Arg et Val.

20. Variant selon la revendication 19, caractérisé en ce que X¹⁰ est Val ou Lys.

21. Variant selon la revendication 1, caractérisé en ce que X¹¹ est Ser ou Gly.

22. Variant selon la revendication 1, caractérisé en ce que X¹² est un résidu d'acide aminé choisi dans le groupe constitué de Gly, Met, Gln, Glu, Leu, Arg, Lys, Pro et Asn.

23. Variant selon la revendication 22, caractérisé en ce que X¹² est Ala ou Leu.

24. Variant selon la revendication 1, caractérisé en ce que X¹³ est Ala ou Gly.

25. Variant selon la revendication 1, caractérisé en ce que X¹⁴ est un résidu d'acide aminé choisi dans le groupe constitué de Lys, Asn et Asp.

26. Variant selon la revendication 25, caractérisé en ce que X¹⁴ est Lys ou Asn.

27. Variant selon la revendication 1, caractérisé en ce que X¹⁵ est un résidu d'acide aminé choisi dans le groupe constitué de Val, Tyr, Asp, Glu, Thr, Gly, Leu, Ser, Ile, Gln, His, Asn, Pro, Phe, Met, Ala, Arg, Trp et Lys.

28. Variant selon la revendication 27, caractérisé en ce que X¹⁵ est Lys ou Glu.

29. Variant selon la revendication 1, caractérisé en ce que X¹⁶ est Gly.

30. Variant selon la revendication 1, caractérisé en ce que X¹ est Gly-Pro et X¹⁶ est Gly.

31. Variant selon la revendication 1, comprenant la séquence d'acides aminés suivante

32. Construction d'ADN comprenant une séquence d'ADN codant pour un variant humain inhibiteur de la protéase du type Kunitz selon l'une quelconque des revendications 1-31.

33. Vecteur d'expression recombinant comprenant une construction d'ADN selon la revendication 32.

34. Cellule contenant une construction d'ADN selon la revendication 32 ou un vecteur d'expression selon la revendication 33.

35. Procédé de production d'un variant humain inhibiteur de la protéase du type Kunitz selon l'une quelconque des revendications 1-31, le procédé comprenant la culture d'une cellule selon la revendication 34 dans des conditions favorables à l'expression de la protéine, et la récupération de la protéine résultante à partir de la culture.

36. Composition pharmaceutique comprenant un variant humain inhibiteur de la protéase du type Kunitz selon l'une quelconque des revendications 1-31 et un support ou un excipient pharmaceutiquement acceptable.

37. Composition selon la revendication 36, comprenant en outre de l'héparine.

38. Utilisation du domaine III inhibiteur de la protéase du type Kunitz humain de TFPI ou un variant de celui-ci selon l'une quelconque des revendications 1-31, pour la préparation d'un médicament pour la prévention ou le traitement de maladies ou d'infections associées à la protéolyse pathologique.


### SEQUENCE LISTING

(1) GENERAL INFORMATION:
   (i) APPLICANT:
      (A) NAME: Novo Nordisk A/S
      (B) STREET: Novo Alle
      (C) CITY: Bagsvaerd
      (E) COUNTRY: Denmark
      (F) POSTAL CODE (ZIP): DK-2880
      (G) TELEPHONE: +45 4444 8888
      (H) TELEFAX: +45 4449 3256
      (I) TELEX: 37304
   (ii) TITLE OF INVENTION: A Human Kunitz-type Protease Inhibitor Variant
   (iii) NUMBER OF SEQUENCES: 2
   (iv) COMPUTER READABLE FORM:
      (A) MEDIUM TYPE: Floppy disk
      (B) COMPUTER: IHM PC compatible
      (C) OPERATING SYSTEM: PC-DOS/MS-DOS
      (D) SOFTWARE: PatentIn Release #1.0, Version #1.25 (EPO)
(2) INFORMATION SEQ ID NO: 1:
   (i) SEQUENCE CHARACTERISTICS:
      (A) LENGTH: 57 amino acids
      (B) TYPE: amino acid
      (C) STRANDEDNESS: single
      (D) TOPOLOGY: linear
   (ii) MOLECULE TYPE: protein
   (vi) ORIGINAL SOURCE:
      (A) ORGANISM: synthetic
   (xi) SEQUENCE DESCRIPTION: SEQ ID NO: 1:
(2) INFORMATION FOR SEQ ID NO: 2:
   (i) SEQUENCE CHARACTERISTICS:
      (A) LENGTH: 58 amino acids
      (B) TYPE: amino acid
      (D) TOPOLOGY: linear
   (ii) MOLECULE TYPE: protein
   (vi) ORIGINAL SOURCE:
      (A) ORGANISM: synthetic
   (xi) SEQUENCE DESCRIPTION: SEQ ID NO: 2:
   Gly Pro Ser Trp Cys Leu Thr Pro Ala Asp Arg Gly Pro Cys Lys Ala
   1 5 10 15
   Arg Ile Ile Arg Phe Tyr Tyr Asn Ser Val Ile Gly Lys Cys Arg Pro
   20 25 30
   Phe Val Tyr Gly Gly Cys Arg Ala Lys Glu Asn Asn Phe Lys Ser Lys
   35 40 45
   Gln Glu Cys Leu Arg Ala Cys Lys Lys Gly
   50 55